# EUROPEAN PATENT APPLICATION

(11) **EP 2 306 351 A1**
(43) Date of publication of application: **06.04.2011**
(21) Application number: 09171104.4
(22) Date of filing: 23.09.2009
(51) Int. Cl.: G06F 19/00

(54) **Health management device**

(71) Applicant: EPS Bio Technology Corp., Hsin-Chu City 300 (TW)
(72) Inventor: Yuan, Wen Ching, Hsin-chu City (TW)
(74) Representative: Lang, Christian

(57) **Abstract**

A health management device (100, 200, 300) is disclosed in embodiments of the present invention. The health management device (100, 200, 300) comprises at least one sensor (101, 201, 301), a microprocessor (102, 202, 302) and a memory unit (103, 203, 303). The sensor (101, 201, 301) measures the status of a target and generates a sensing signal (S). The microprocessor (102, 202, 302), coupling to the sensor (101, 201, 301), processes the sensing signal (S) and generates a data signal (I). The memory unit (103, 203, 303), coupling to the microprocessor (102, 202, 302), generates a measurement data (DT) according to the data signal (I) and stores the measurement data (DT). Wherein, at least one health management software (103a, 203a, 303a) is embedded in the memory unit (103, 203, 303) and computes the data signal (I) to obtain the measurement data (DT).

## Description

### Background of the invention

### (a) Field of the Invention

The invention relates to a health management device, particularly to a health management device for recording the physiology information of a user for a long period of time and making statistics of the recorded information to be easily operable for the user in his own residence.

### (b) Description of the Related Art

Currently, a general physiology measurement device, such as sphygmomanometer, blood sugar meter, etc., only shows the currently measured physiology parameter. However, when the result of the measurement is normal, it does not guarantee that the body function is well. Sometimes, pathological changes have the incubation period. Thus, it is not easy to be aware of the hidden risk for a user and the health status cannot be effectively managed and improved. Besides, a general measurement device having on-line and data management functions requires additional cost and installation effort for the management software and driver that causes operation barriers.

### BRIEF SUMMARY OF THE INVENTION

Therefore, in order to solve the above-mentioned problem, one object of the invention is to provide a health management device for recording the physiology information within a predetermined period of time.

One object of the invention is to provide a health management device operable in a computer system without installing additional software in the computer.

One object of the invention is to provide a health management device, storing the detected information in a swappable/swap memory unit.

One embodiment of the invention provides a health management device, comprising at least one sensor, a microprocessor and a memory unit. The sensor measures physiology information and generates a sensing signal. The microprocessor, coupling to the sensor, processes the sensing signal and generates a data signal. The memory unit, coupling to the microprocessor, generates a measurement data according to the data signal and stores the measurement data. At least one health management software is embedded in the memory unit and is executed on a compatible platform to compute and analyze the data signal to obtain the measurement data according to the operation by a user.

Another embodiment of the invention provides a health management device, comprising at least one sensor, a microprocessor, a memory unit, and a transmission interface. The sensor measures physiology information and generates a sensing signal. The microprocessor, coupling to the sensor, processes and analyzes the sensing signal and generates a data signal. The memory unit, coupling to the microprocessor, generates a measurement data according to the data signal and stores the measurement data. At least one health management software is embedded in the memory unit and is executed on a compatible platform to compute and analyze the data signal to obtain the measurement data according to the operation by a user. The sensing signal comprises one of the information selected from the group consisting of the following or combination thereof: blood sugar data, blood pressure data, and body weight data. The transmission interface couples to the memory unit for outputting the measurement data.

One other embodiment of the invention provides a health management device, comprising at least one sensor, a microprocessor, a first transmission interface, a memory unit, and a second transmission interface. The sensor measures physiology information and generates a sensing signal. The microprocessor, coupling to the sensor, processes the sensing signal and generates a data signal. The first transmission interface couples to the microprocessor for outputting the data signal. The memory unit, coupling to the transmission interface, generates a measurement data according to the data signal and stores the measurement data. The second transmission interface couples to the memory unit for outputting the measurement data. At least one health management software is embedded in the memory unit and is executed on a compatible platform to compute and analyze the data signal to obtain the measurement data according to the operation by a user. The sensing signal comprises one of the information selected from the group consisting of the following or combination thereof: blood sugar data, blood pressure data, and body weight data.

The health management device according to the invention utilizes the embedded health management software to record the physiology parameters for a long period of time and make statistics of these data and can be easily operable in the other computer system. Therefore, the health status of a user can be effectively managed and improved.

### Brief description of the drawings

FIG. 1A shows a schematic diagram illustrating the health management device according to one embodiment of the invention.

FIG. 1B and FIG. 1C show a statistical chart generated by the health management device shown in FIG. 1A.

FIG. 2 shows a schematic diagram illustrating the health management device according to one embodiment of the invention.

FIG. 3 shows a schematic diagram illustrating the health management device according to one embodiment of the invention.

### DETAILED DESCRIPTION OF THE INVENTION

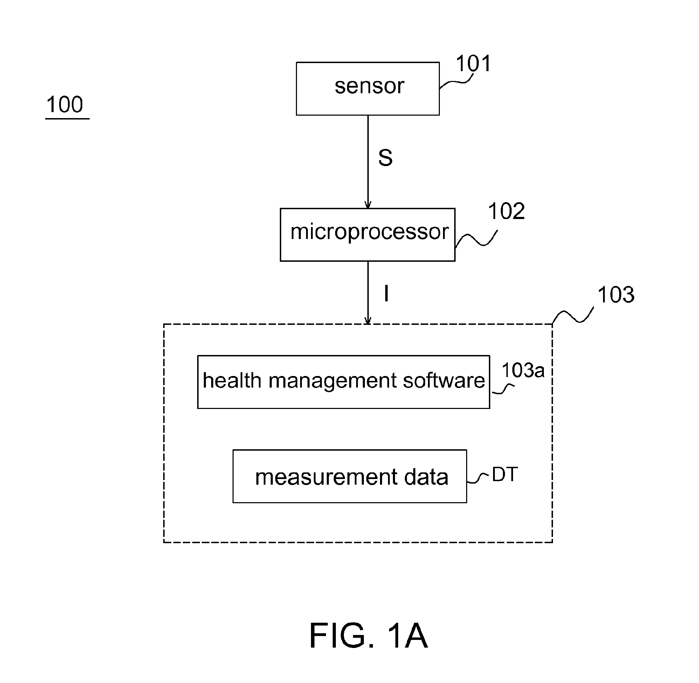

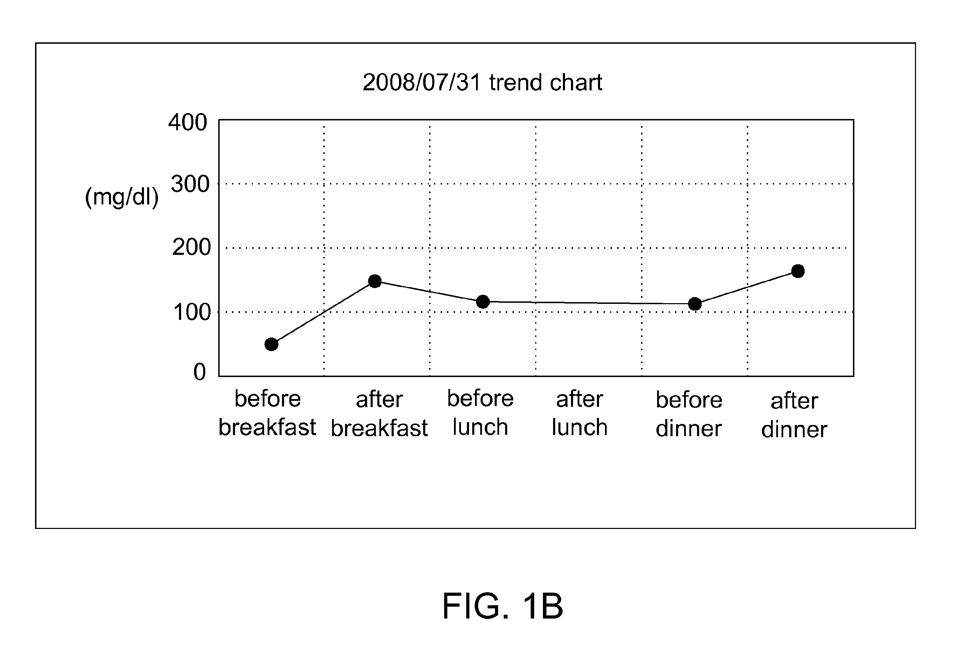

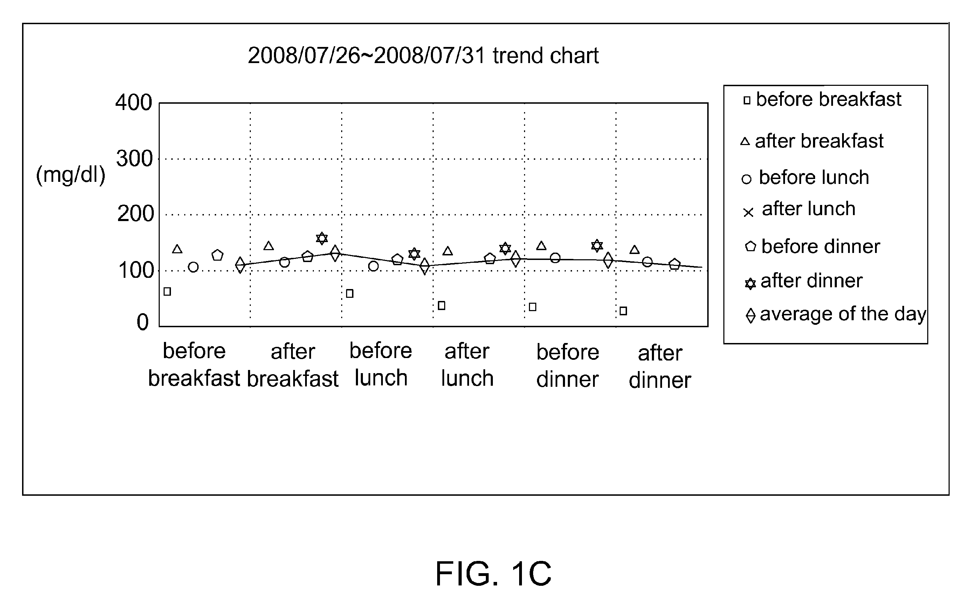

Please refer to FIG. 1A, FIG. 1B and FIG. 1C. FIG. 1A shows a schematic diagram illustrating the health management device according to one embodiment of the invention while FIG. 1B and FIG. 1C show a statistical chart generated by the health management device shown in FIG. 1A.

As shown in FIG. 1A, the health management device 100 comprises at least one sensor 101, a microprocessor 102, and a memory unit 103.

The sensor 101 is used to measure physiology information (status) of a user to generate a sensing signal S and transmit the sensing signal S to the microprocessor 102. The microprocessor 102 couples to the sensor 101 for processing the sensing signal S and then generating a data signal I. It should be noted that the sensing signal S can include blood sugar data, blood pressure data, or body weight data, or any combination of the above.

The memory unit 103 couples to the microprocessor 102 and has at least one embedded health management software 103a. The health management software 103a is executed on a compatible platform to compute and analyze the data signal I to obtain the measurement data DT according to the operation by a user. The health management software 103a is proprietary native software (for example: win32) or cross-platform software (for example: java or java script) and the compatible platform is determined by the setting of the designer and can be any current platform or the platform to be developed in the future.

According to at least a preset rule, the health management software 103a performs one task selected from the group consisting of the following or combination thereof: data statistics, management, and operation. For example, the preset rule can be calculating all of the historical data signals I in the memory unit 103, calculating the historical data signals I within N days where N is more than 0 and less than infinity, or a blood sugar calculation equation. But, the invention is not limited to the above mentioned examples. The measurement data DT calculated by the health management software 103a comprises the average value, the trend chart, and the physiology parameter log. The user can record the blood sugar value before and after each of the three meals everyday and calculate the average of the blood sugar value for each period of time and the percentage that is beyond the normal blood sugar value via the health management software 103a. Referring to the following Table 1, the blood sugar values for each period of time from July 26, 2008 to July 31, 2008 are shown. The health management software 103a tables the measurement data DT for the user to understand his blood sugar value to be at either high or low level.

For example, on July 31, 2008, the blood sugar values for each period of time are 71, 137, 114, and 110 separately, and the average calculated by the health management software 103a is 108.

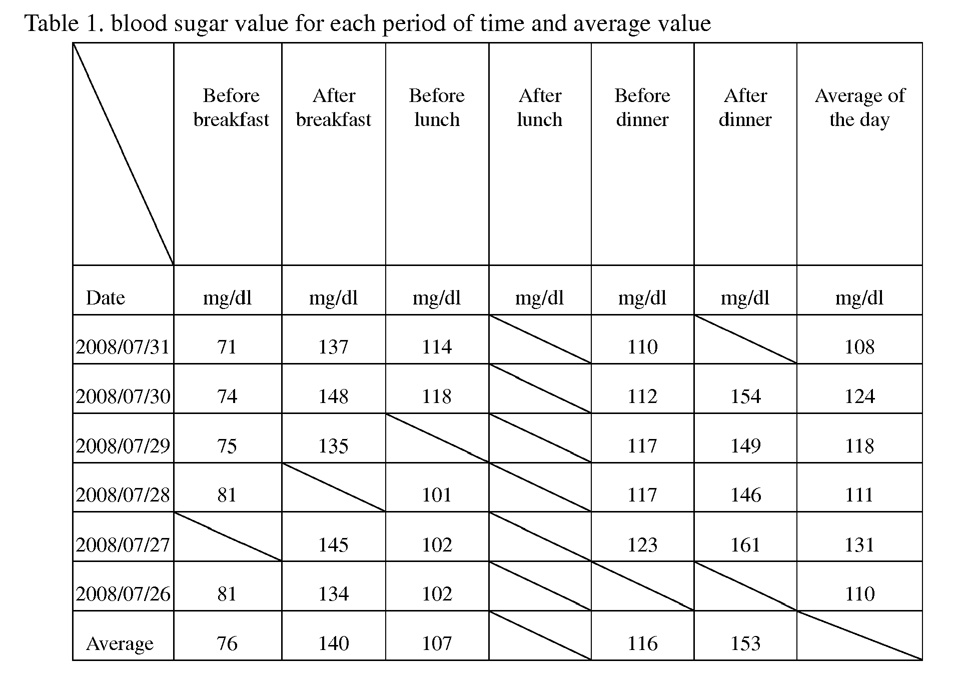

In addition, in order to conveniently monitor the blood sugar of a user, the health management device 100 plots the trend chart and the average trend chart of the blood sugar values for each period of time for each day. The trend chart or statistical chart demonstrates the data in a way that the user can easily realize his body status.

It should be noted that FIG. 1B and FIG. 1C show the blood sugar values as an example but the invention is not limited to this example. Although this embodiment uses the blood sugar value as an example, the operating principle of the other physiology parameter, such as blood pressure, pulse rate, body fat, body weight, etc., is in the same manner and thus its details will not be described hereafter.

As described in the above, finally the measurement data DT is stored in the memory unit 103. The memory unit 103 can be implemented by a flash memory or other current or future storage device.

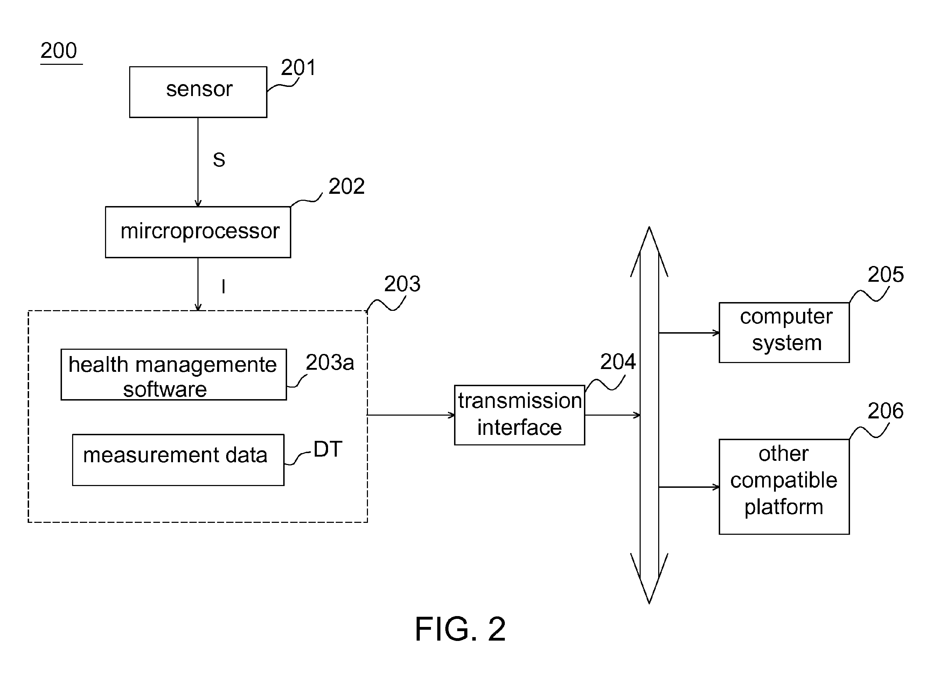

FIG. 2 shows a schematic diagram illustrating the health management device 200 according to another embodiment of the invention. The difference between the health management device 100 and the health management device 200 is that the memory unit 203 is a swappable/swap device. Since the health management software 203a is built in the memory unit 203, when the memory unit 203 is unplugged from the health management device 200, a transmission interface 204, such as universal serial bus (USB), can transmit the measurement data DT to a computer system 205 or one other compatible platform 206. The transmission interface 204 includes at least one mass storage class, such as external hard disc, flash drive, card reader, etc. The additional driver is not required to be installed in the computer system 205 or the compatible platform 206. The health management software 103a can be operated or accessed directly from the memory unit 203 by the computer system 205 or the compatible platform 206. It is convenient for a user to operate in the computer at home. Moreover, the measured information can be provided to the physicians or as a reference for interrogation enquiry or therapy. The long-term records of the physiology parameter can assist in the effect of the whole therapy.

Furthermore, since the memory unit 203 is a swappable/swap device, the user can replace the memory unit 203 for different physiological needs. For example, at the beginning a user purchases a health management device and a memory unit with the embedded blood sugar management software. After that, if the user wants to measure body fat and then manage it, the user can just purchase another memory unit with the embedded body fat management software. Unlike the current product, there is no need to purchase many measurement devices for measuring various types of physiology information. Only one health management device is needed. Thus measuring and managing various types of physiology information can be achieved by utilizing different memory units. Therefore, the user can have the flexibility in utilizing the health management device and obtain the health monitoring result with better quality by lower cost.

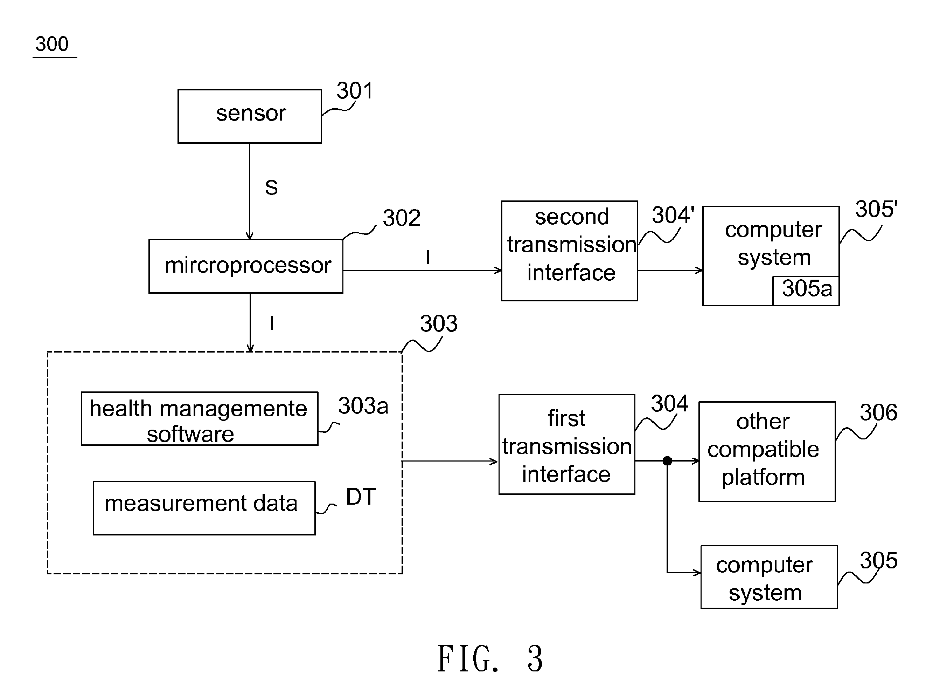

FIG. 3 shows a schematic diagram illustrating the health management device 300 according to another embodiment of the invention. The difference between the health management device 200 and the health management device 300 is that the health management device 300 comprises two transmission interfaces 304 and 304'. During operation, the sensor 301 generates a sensing signal S after measuring physiology information of a user and then transmits the sensing signal S to the microprocessor 302. The data signal I issued by the microprocessor 302 is outputted to a computer system 305' via the second transmission interface 304' and stored by the storage device 305a of the computer system 305'. The storage device 305a can be implemented by a flash memory, hard disk (HD), or other current or future storage device. In addition, the first transmission interface 304 and the second transmission interface 304' can be USBs and the second transmission interface 304' comprises at least one mass storage class, such as hard external disc, flash drive, card reader, etc. By such allocation, the health management device 300 according to this embodiment is more flexible in operation. Thus, it is convenient in use.

In conclusion, the health management device according to the invention utilizes the embedded health management software to make long-term records and statistics according to the need of a user. The health management software can be easily executed on the other computer system. Therefore, the health status of the user can be effectively managed and improved.

## Claims

1. A health management device (100), comprising:
at least one sensor (101) for measuring physiology information and generating a sensing signal (S);
a microprocessor (102) coupling to the sensor (101) for processing the sensing signal (S) and generating a data signal (I); and
a memory unit (103) coupling to the microprocessor (102) for generating a measurement data (DT) according to the data signal (I) and for storing the measurement data (DT);
wherein at least one health management software (103a) is embedded in the memory unit (103) and is executed on at least a compatible platform to compute and analyze the data signal (I) to obtain the measurement data (DT) according to the operation by a user.

2. The device according to claim 1, wherein the memory unit (103) is a swappable/swap device or a flash memory.

3. The device according to claim 1, wherein the health management software (103a) is proprietary native software or cross-platform software and the health management software (103a) performs one task selected from the group consisting of the following or combination thereof: data statistics, management, and operation.

4. The device according to claim 1, wherein the measurement data (I) is selected from the group consisting of the following or combination thereof: the average of measurement and the trend chart of measurement.

5. The device according to claim 1, further comprising: a transmission interface that is a means of communication among the sensor, the microprocessor, and the memory unit.

6. The device according to claim 1, wherein measurement data (DT) is outputted via a transmission interface, in particular via a transmission interface to a computer system or a compatible platform, or the transmission interface is a universal serial bus (USB) transmission interface and the transmission interface comprises at least one mass storage class.

7. A health management device (200), comprising:
at least one sensor (201) for measuring physiology information and generating a sensing signal (S);
a microprocessor (202) coupling to the sensor (201) for processing and analyzing the sensing signal (S) and generating a data signal (I);
a memory unit (203) coupling to the microprocessor (202) for generating a measurement data (DT) according to the data signal (I) and for storing the measurement data (DT) wherein at least one health management software (203a) is embedded in the memory unit (203) and is executed on a compatible platform to compute and analyze the data signal (I) to obtain the measurement data (DT) according to the operation by a user; and
a transmission interface (204) coupling to the memory unit (203) for outputting the measurement data (I).

8. The device according to claim 7, wherein the sensing signal (S) comprises one of the information selected from the group consisting of the following or combination thereof: blood sugar data, blood pressure data, and body weight data.

9. The device according to claim 7, wherein the memory unit (203) is a swappable/swap device or a flash memory, or the health management software (203a) is a proprietary native software or cross-platform software.

10. The device according to claim 7, wherein the measurement data (DT) is either the average value calculated or the trend chart plotted by measuring the physiology information of a user for a plurality of times within a predetermined period via the health management software (203a) or the combination of the average and the trend chart.

11. The device according to claim 7, wherein the transmission interface (204) is a universal serial bus (USB) transmission interface and the transmission interface (204) comprises at least one mass storage class, or measurement data (DT) is outputted via a transmission interface (204) to a computer system (205) or a compatible platform (206).

12. A health management device (300), comprising:
at least one sensor (301) for measuring physiology information and generating a sensing signal (S);
a microprocessor (302) coupling to the sensor (301) for processing and analyzing the sensing signal (S) and generating a data signal (I);
a second transmission interface (304') coupling to the microprocessor for outputting the data signal (I);
a memory unit (303) coupling to the transmission interface for generating a measurement data (DT) according to the data signal (I) and for storing the measurement data (DT) ; and
a first transmission interface (304) coupling to the memory unit (303) for outputting the measurement data (DT);
wherein at least one health management software (303a) is embedded in the memory unit (303) and is executed on a compatible platform to compute and analyze the data signal (I) to obtain the measurement data (DT) according to the operation of a user.

13. The device according to claim 12, wherein the sensing signal (S) comprises one of the information selected from the group consisting of the following or combination thereof: blood sugar data, blood pressure data, and body weight data.

14. The device according to claim 12, wherein the memory unit (303) is a swappable/swap device or a flash memory, or the health management software (303a) is a proprietary native software or cross-platform software.

15. The device according to claim 12, wherein the measurement data (DT) is selected from the group consisting of the following or combination thereof: the average of measurement, the trend chart of measurement, measurement data (DT) is outputted via the first transmission interface (304) to a computer system (305) or a compatible platform (306), or the first and the second transmission interfaces (304, 304') are universal serial bus (USB) transmission interfaces and the second transmission (304') interface comprises at least one mass storage class.
